# EUROPÄISCHE PATENTSCHRIFT

(11) **EP 1 225 848 B1**
(45) Veröffentlichungstag und Bekanntmachung des Hinweises auf die Patenterteilung: **15.06.2005**
(21) Anmeldenummer: 00981217.3
(22) Anmeldetag: 30.10.2000
(51) Int. Cl.: A61F 2/28

(54) **VERFAHREN ZUR HERSTELLUNG EINES KNOCHENIMPLANTATS**
METHOD OF MANUFACTURING A BONE IMPLANT
PROCEDE DE FABRICATION D'UN IMPLANT OSSEUX

(30) Priorität: 02.11.1999 DE 19952550
(43) Veröffentlichungstag der Anmeldung: 31.07.2002
(73) Patentinhaber: Tutogen Medical GmbH, 91077 Neunkirchen am Brand (DE)
(72) Erfinder: SCHÖPF, Christoph, 91096 Möhrendorf (DE); KOSCHATZKY, Karl, 91058 Erlangen (DE); KALAS, Rolf-Dieter, 34320 Söhrewald 1 (DE); LÖWEL, Matthias, 90408 Nürnberg (DE)
(74) Vertreter: Manitz, Finsterwald & Partner GbR
(86) Internationale Anmeldenummer: PCT/EP2000/010673
(87) Internationale Veröffentlichungsnummer: WO 2001/032107

(56) Entgegenhaltungen:
- EP-A- 0 315 283
- EP-A- 0 328 478
- EP-A- 0 472 315
- EP-A- 0 574 098
- EP-A- 0 584 484
- EP-A- 0 834 294
- WO-A-00/74741
- WO-A-94/09722
- WO-A-95/32623
- WO-A-98/14128
- DE-A- 19 543 110
- DE-A- 19 701 778
- DE-A- 19 751 284
- DE-A- 19 952 550
- DE-C- 4 341 367
- FR-A- 2 633 823
- GB-A- 2 093 701
- US-A- 4 436 684
- US-A- 5 156 777

## Beschreibung

Die vorliegende Erfindung betrifft ein Verfahren zur Herstellung eines Knochenimplantats zum Einbau einer künstlichen Gelenkpfanne in einen schadhaften Knochen.

Die Lockerung künstlicher Gelenke, insbesondere künstlicher Hüftgelenke, ist regelmäßig mit einem Verlust von Knochensubstanz beim Patienten verbunden. Eine spezielle Form dieses Substanzverlustes ist der konzentrische Knochenschwund rund um die normalerweise halbkugelförmige, künstliche Hüftgelenkpfanne. Die Rekonstruktion des Knochendefektes gestaltet sich häufig sehr schwierig und aufwendig, insbesondere wenn der Knochendefekt an der Pfannenrückseite die innere Kortikalis einschließt. Gegenwärtig wird mit großem technischen und zeitlichen Aufwand versucht, den Knochendefekt durch Verwendung von Metallimplantaten zu überbrücken, um der Wechselpfanne eine stabile, großflächig am gesunden Knochen abgestützte Unterlage zu bieten.

Die gegenwärtig angewendeten Methoden verwenden dabei eine Kombination aus speziellen Metallimplantaten und Knochenblöcken, Scheiben und Granulat, deren benötigte Größe und Menge erst intraoperativ bestimmt werden kann. Hierdurch wird die Operation erheblich verlängert mit allen sich daraus ergebenen Nachteilen für den Patienten.

Aus der EP 0 834 294 A1 sind Distanzstücke für in Knochen zementierbare Prothesenteile bekannt, die aus ausgehärtetem Knochenzement bestehen.

In der DE 195 43 110 A1 ist ein steriles Knochenmaterial nativen Ursprungs, beispielsweise Spongiosa-Knochen, für die Transplantation beschrieben, das im wesentlichen frei von Fett, Bindegewebe und Knorpelmasse ist.

Gemäß einem beispielsweise aus der US-A-4 436 684 bekannten Verfahren zur Herstellung eines Knochenimplantats zum Einbau einer künstlichen Gelenkpfanne in einen schadhaften Knochen wird von dem schadhaften Körperknochen des Patienten ein Computertomogramm angefertigt und mit dessen Hilfe ein künstliches Modell des schadhaften Körperknochens hergestellt.

Insbesondere kann in den Bereich des knochendefektes am Modell eine aushärtbare Abformmasse eingefüllt werden, so dass die Abformmasse zu einem Formstück ausgehärtet wird.

Es ist die Aufgabe der vorliegenden Erfindung, ein Verfahren zur Herstellung eines Knochenimplantats zu schaffen mit dem die Planung und Operation eines Knochendefekts vereinfacht werden kann.

Die Lösung dieser Aufgabe erfolgt durch ein Verfahren mit den Merkmalen des Anspruchs 1.

Ein gemäß Anspruch 1 gewonnenes Knochenimplantat fügt sich paßgenau in den Knochendefekt ein und erfüllt so die Anforderungen an eine stabile, großflächige Abstützung am gesunden Knochen. Gleichzeitig können mit diesem Knochenimplantat eventuelle Pfannenhinterwanddefekte stabil und zuverlässig verschlossen werden.

Vorteilhafte Ausführungsformen der Erfindung sind durch die Beschreibung, die Figuren sowie die Unteransprüche beschrieben.

Bei der Bildung einer Aufnahme für eine Gelenkpfanne kann die geplante Wechselpfanne einer Endoprothese, oder ein Modell davon, in die Abformmasse eingesetzt oder eingedrückt werden, um die Außenkontur der Gelenkpfanne oder des Modells in der Abformmasse abzubilden.

Auch ist es vorteilhaft, Mittel zur Fixation vorzusehen, d.h. in der Abformmasse abzubilden. Dazu kann die günstigste Verlaufsrichtung von Befestigungselementen wie Schrauben oder Stiften durch Eindrücken von Stiften in die Abformmasse festgelegt werden. Ebenso kann die gewünschte oder erforderliche Länge dieser Elemente vorab festgelegt werden.
Durch Vorbohren der Kanäle sitzen dann die Befestigungselemente automatisch korrekt.

Erfindungsgemäß wird ein aus spongiosem Knochen bestehendes Standardprodukt vorgesehen, das seine biomechanischen Eigenschaften beibehält, umbaufähig ist und gleichzeitig zur Verankerung einer Gelenkpfanne dienen kann. Der Grundkörper läßt sich in kurzer Zeit in den schadhaften Knochen einsetzen, beispielsweise durch Presspassung, woraufhin die Gelenkpfanne der Endoprothese in den Hohlraum des Grundkörpers eingesetzt werden kann.

Durch die Verwendung eines solchen standardisierten Zwischenteils aus natürlicher Spongiosa, das gewissermaßen als "Adapter" eingesetzt wird, läßt sich die Planungs- und Operationszeit erheblich verkürzen, da darauf verzichtet werden kann, eine Vielzahl von Einzelmaßnahmen zur Behebung des Knochendefektes und zur Verankerung der Gelenkschale durchzuführen.

Bevorzugt wird das Knochenimplantat aus bovinem Knochen hergestellt.

Nachfolgend wird die vorliegende Erfindung beispielhaft anhand vorteilhafter Ausführungsformen und unter Bezugnahme auf die beigefügten Zeichnungen beschrieben. Es zeigen:
- Fig. 1: eine perspektivische Ansicht einer Ausführungsform eines Knochenimplantats;
- Fig. 2: eine Draufsicht auf das Knochenimplantat von Fig. 1; und
- Fig. 3: eine Draufsicht auf eine weitere Ausführungsform eines Knochenimplantats.

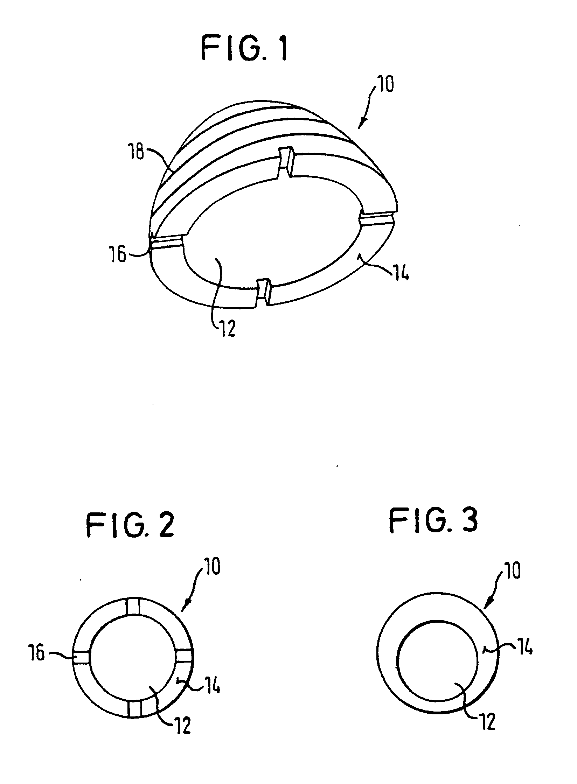

Bei dem in Fig. 1 dargestellten Knochenimplantat handelt es sich um eine etwa halbkugelförmige Schale 10 aus konservierter, natürlicher Spongiosa, die einen halbkugelförmigen Hohlraum 12 aufweist. Die Außenkontur des Knochenimplantats 10 sowie die Innenkontur des Hohlraums 12 ist somit halbkugelförmig oder zumindest angenähert halbkugelförmig. Die kreisrunde Öffnung des Hohlraums 12 ist von einer umlaufenden Stirnfläche 14 umgeben, die bei dem dargestellten Ausführungsbeispiel mehrere radial verlaufenden Vertiefungen 16 zum Ansetzen eines Setzwerkzeugs aufweist.

An der halbkugelförmigen Außenfläche des Knochenimplantats ist eine Außenstruktur 18 vorgesehen, mit der das Knochenimplantat in dem schadhaften Knochen verankert werden kann, indem ein Werkzeug in die Vertiefungen 16 eingesetzt wird. Die Außenstruktur 18 kann als Gewinde ausgebildet sein.

Fig. 2 zeigt eine Draufsicht auf das Knochenimplantat von Fig. 1. Wie zu erkennen ist, ist die Mitte der Öffnung des Hohlraums 12 konzentrisch zur Mitte des Grundkörpers 10 angeordnet.

Bei der in Fig. 3 in Draufsicht dargestellten Ausführungsform ist demgegenüber die Mitte des Hohlraums 12 versetzt zur Mitte des Grundkörpers 10 angeordnet, d. h. der Hohlraum 12 ist exzentrisch angeordnet. Hierdurch kann die Position des Hohlraums durch Verdrehen variiert werden. Ebenso ist bei dieser Ausführungsform, die durch eine Preßpassung in dem schadhaften Knochen verankert wird, kein Außengewinde vorgesehen. Entsprechend fehlen auch die Vertiefungen 16.

Die Außendurchmesser der dargestellten Knochenimplantate können in einem Bereich von etwa 30 mm bis 70 mm variieren. Die Innendurchmesser sind jeweils an handelsübliche Außendurchmesser künstlicher Gelenkschalen von Endoprothesen angepaßt.

Die vorliegende Erfindung erlaubt die Vorbereitung des Implantatlagers mit handelsüblichen Raspartorien durch Ausraspeln der bindegewebartigen Strukturen bis auf den gesunden Knochen. Durch Wahl der passenden Knochenschalc ist eine Implantation durch Presspassung möglich, wobei gleichzeitig Pfannenhinterwanddefekte stabil verschlossen werden. In der in den schadhaften Knochen eingesetzten Knochenschale kann nun die Wechselpfanne mit Presspassung, durch Einzementieren oder durch Verschrauben eingepaßt werden. Eine Verschraubung kann gleichzeitig auch der Verankerung der Knochenschale dienen.

Ausgangsmaterial für das Knochenimplantat ist tierischer Knochen von ausreichend großen Tieren, vorzugsweise Rindern, aber auch anderen ähnlich großen Tieren, die unter kontrollierten Bedingungen gehalten werden können.

Zur Entfernung der Antigenität wird der Knochen einer osmotischen Behandlung unterzogen. Weiterhin wird zur Denaturierung löslicher Proteine eine oxidative Behandlung durchgeführt. Zur Optimierung der Virusinaktivierung kann eine pH-Absenkung auf pH 3 erfolgen oder eine Behandlung mit Natronlauge oder einer anderen DNA/RNA zerstörenden Substanz. Der Wasserentzug erfolgt durch organische Lösungsmittel, beispielsweise Azeton. Die abschließende Sterilisation erfolgt durch energiereiche Strahlung, vorzugsweise Gammastrahlung mit einem Dosismaximum von 25 kGy. Der so behandelte Knochen behält seinen natürlichen Mineral-Kollagen-Verbund und damit seine biomechanischen Eigenschaften und er ist umbaufähig.

Bei einem Verfahren zur Herstellung eines Knochenimplantats für den Austausch von schadhaften Gelenkpfannen wird erfindungsgemäß von dem schadhaften Körperknochen des Patienten zunächst ein Computertomogramm angefertigt, mit dessen Hilfe ein künstliches Modell des schadhaften Körperknochens hergestellt wird. Anschließend wird in den Bereich des Knochendefektes am Modell eine aushärtbare Abformmasse eingefüllt, wobei danach die Abformmasse so geformt wird, daß sie eine Aufnahme für eine künstliche Gelenkpfanne bildet. Hierbei füllt der Chirurg in den Knochendefekt am Modell eine vorzugsweise selbst aushärtende Abformmasse und formt anschließend die Masse nach seinen Wünschen. Die künstliche Gelenkpfanne, die eingesetzt werden soll, kann an der gewünschten Stelle in die Abformmasse eingedrückt und somit abgeformt werden. Gleichfalls kann bereits die Fixation der Gelenkpfanne vorgeplant werden.

Anschließend läßt man die Abformmasse aushärten und mit Hilfe des ausgehärteten Formstückes wird aus einem ausreichend großen Stück konservierter Spongiosa natürlichen Ursprungs (beispielsweise durch Kopierfräsen) ein Knochenimplantat durch Fräsen hergestellt, welches dem ausgehärteten Formstück entspricht. Das so gewonnene Implantat fügt sich paßgenau in den Defekt ein, und erfüllt die Anforderungen an eine stabile, großflächige Abstützung am gesunden Knochen. Gleichzeitig können mit diesem Knochenimplantat eventuelle Pfannenhintergrunddefekte stabil und zuverlässig verschlossen werden.

## Patentansprüche

1. Verfahren zur Herstellung eines Knochenimplantats zum Einbau einer künstlichen Gelenkpfanne in einen schadhaften Knochen, bei dem von dem schadhaften Körperknochen des Patienten ein Computertomogramm angefertigt wird, mit dessen Hilfe ein künstliches Modell des schadhaften Körperknochens hergestellt wird, wobei
a) in den Bereich des Knochendefektes am Modell eine aushärtbare Abformmasse eingefüllt wird,
b) die Abformmasse so geformt wird, daß sie eine Aufnahme für eine künstliche Gelenkpfanne bildet,
c) die Abformmasse zu einem Formstück ausgehärtet wird, und
d) auf einer Fräsmaschine durch Abtasten des ausgehärteten Formstücks eine Kopie des Formstückes angefertigt wird, die aus einem Stück konservierter Spongiosa gefräst wird.

2. Verfahren nach Anspruch 1,
**dadurch gekennzeichnet, dass**
im Schritt b) eine Gelenkpfanne einer Endoprothese oder ein Modell der Gelenkpfanne in die Abformmasse eingesetzt wird, um die Außenkontur der Gelenkpfanne oder des Modells in der Abformmasse abzubilden.

3. Verfahren nach Anspruch 1,
**dadurch gekennzeichnet, dass**
vor dem Schritt c) an der Abformmasse Mittel zur späteren Fixation des Knochenimplantats an dem schadhaften Körperknochen vorgesehen werden.

## Claims

1. A method for the manufacture of a bone implant for fitting an artificial joint socket into a damaged bone, in which a computer tomogram is made from the damaged body bone of the patient with the aid of which an artificial model of the damaged body bone is produced, wherein
a) a hardenable molding composition is filled into the region of the bone defect at the model;
b) the molding composition is shaped such that it forms a reception for an artificial joint socket;
c) the molding composition is hardened into a molded piece; and
d) a copy of the molded piece is made on a milling machine by scanning the hardened molded piece, said copy being milled from a piece of preserved spongiosa.

2. A method in accordance with claim 1, **characterized in that** in step b) a joint socket of an endoprosthesis or a model of the joint socket is inserted into the molding composition to form the outer contour of the joint socket or of the model in the molding composition.

3. A method in accordance with claim 1, **characterized in that**, before step c), means are provided at the molding composition for the later fixing of the bone implant to the damaged body bone.

## Revendications

1. Procédé de fabrication d'un implant osseux destiné à la mise en place d'une cavité glénoide artificielle dans un os défectueux, dans lequel un tomogramme de l'os corporel défectueux du patient est établi par ordinateur, à l'aide duquel est réalisé un modèle artificiel de l'os corporel défectueux, étant précisé que
a) une masse de conformation durcissable est introduite dans la zone de la défectuosité osseuse du modèle,
b) la masse de conformation est conformée de telle sorte qu'elle forme une réception pour une cavité glénoïde artificielle,
c) la masse de conformation est durcie en une pièce conformée, et
d) une copie de la pièce conformée est réalisée sur une fraiseuse par le balayage de la pièce conformée durcie, laquelle est fraisée dans un morceau de tissu spongieux conservé.

2. Procédé selon la revendication 1,
**caractérisé en ce que**,
dans la phase b), une cavité glénoïde d'une endoprothèse ou un modèle de la cavité glénoide est inséré dans la masse de conformation afin de reproduire le contour extérieur de la cavité glénoide ou du modèle dans la masse de conformation.

3. Procédé selon la revendication 1,
**caractérisé en ce que**,
avant la phase c), des moyens sont prévus sur la masse de conformation pour la fixation ultérieure de l'implant osseux sur l'os corporel défectueux.
